## Europäisches Patentamt

## European Patent Office

## Office européen des brevets

(19)

(11) Veröffentlichungsnummer: **0 106 945**
**B1**

(12) **EUROPÄISCHE PATENTSCHRIFT**

(45) Veröffentlichungstag der Patentschrift:
**15.10.86**

(51) Int. Cl.⁴: **A 61 F 2/30**

(21) Anmeldenummer: **83107330.9**

(22) Anmeldetag: **26.07.83**

(54) Verankerungsschaft für die Verankerung von Knochenimplantaten.

(30) Priorität: **15.10.82  CH 6018/82**

(43) Veröffentlichungstag der Anmeldung:
**02.05.84 Patentblatt 84/18**

(45) Bekanntmachung des Hinweises auf die Patenterteilung:
**15.10.86 Patentblatt 86/42**

(84) Benannte Vertragsstaaten:
**AT DE FR IT**

(56) Entgegenhaltungen:
**DE-A-2 732 923**
**FR-A-2 169 945**
**GB-A-2 024 631**

(73) Patentinhaber: **GEBRÜDER SULZER AKTIENGESELLSCHAFT, Zürcherstrasse 9, CH-8401 Winterthur (CH)**

(72) Erfinder: **Frey, Otto, Walrütistrasse 56, CH- 8404 Winterthur (CH)**

(74) Vertreter: **Dipl.- Ing. H. Marsch Dipl.- Ing. K. Sparing Dipl.- Phys.Dr. W.H. Röhl Patentanwälte, Rethelstrasse 123, D-4000 Düsseldorf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 106 945 B1

# Beschreibung

Die Erfindung betrifft einen Verankerungsschaft für die Verankerung von Knochenimplantaten mit Hilfe von Knochenzement, welcher Schaft sich gegen sein freies Ende allseitig und stetig verjüngt und mindestens auf Teilen der Schaftoberfläche mit einer Oberflächenstruktur versehen ist.

Um die Haftung zwischen dem Schaft einer Endoprothese und dem ihn umgebenden Knochenzementbett zu verbessern, ist es bekannt, die Oberfläche des Implantats mindestens auf Teilen der Schaftoberfläche mit einer Strukturierung zu versehen (siehe z.B. CH-A- 547 631). Es hat sich gezeigt, dass bei Schäften mit einer der bekannten Oberflächenstrukturierung infolge des Schrumpfens des Zementbettes in Durchmesser und Umfang die Haftung zwischen dem Knochenzement und dem Verankerungsschaft unter Umständen nicht immer mit der erforderlichen Sicherheit gewährleistet ist; dies führt im Laufe der Zeit zu Relativbewegungen des Schaftes in Zementbett, wodurch dieses "zermahlen" wird, und der Abrieb zwischen die Gleitflächen des Gelenkes gelangen kann.

Aufgabe der Erfindung ist, unter Beachtung der auftretenden Schrumpfung des Knochenzements die Haftung zwischen Implantat und Knochenzement durch eine Struktur auf der Schaftoberfläche weiter zu verbessern; dabei ist zu beachten, dass die Verankerung des Schaftes im Zementbett so sein muss, dass sie - falls eine Reoperation notwendig werden sollte - jederzeit wieder gelöst werden kann. Diese Aufgabe wird mit der Erfindung dadurch gelöst, dass als Oberflächenstruktur eine Anzahl von torbogenartigen, im wesentlichen in Richtung der Schaftlängsachse verlaufenden Vertiefungen mit sich von distal nach proximal konisch verjüngenden Seitenflanken schuppenartig verteilt angeordnet ist.

Die Ausbildung der Struktur als Vertiefungen bewirkt, dass der schrumpfende Knochenzement in die Vertiefungen hinein und nicht von der Oberfläche des Schaftes weg schwindet. Weiterhin ermöglicht der konische Verlauf der Begrenzung der Vertiefungen eine selbstfestigende Wirkung, wenn die Prothese tiefer in den Knochen einzudringen sucht, wobei es zu einer Verdichtung des Knochenzementbettes kommt. Gleichzeitig erleichtert diese konische Form eine Herausnahme des Schaftes bei Reoperationen.

Vorteilhafterweise können die Vertiefungen distal stufenlos in die Schaftoberfläche übergehen und ihre Talsohlen parallel zur Schaftlängsachse verlaufen. Mit diesen beiden zusätzlichen Massnahmen wird zum einen das Eindringen von Knochenzement in die Vertiefungen erleichtert; zum anderen kann dadurch bei Reoperationen das Zementbett weitgehend unbeschädigt erhalten werden.

Um eine Vorstellung der Abmessungen der schuppenartig - d.h. in den einzelnen Reihen versetzt zueinander - angeordneten Vertiefungen zu geben, sei erwähnt:

Bei einer Femurkopfprothese, deren Schaft im allgemeinen eine Länge von etwa 10 bis 25 cm und eine Breite von 1 bis 4 cm hat, beträgt die Höhe einer Vertiefung beispielsweise etwa 5 mm, die Breite des Torbogens an seinem Fuss etwa 3 bis 5 mm und die Tiefe im Scheitel des Bogens weniger als 2 mm.

Im folgenden wird die Erfindung anhand eines Ausführungsbeispiels im Zusammenhang mit der Zeichnung näher erläutert.

Fig. 1 zeigt eine Seitenansicht eines erfindungsgemässen strukturierten Schaftes einer Femurkopfprothese;

Fig. 2 ist im grösseren Maßstab ein Schnitt II-II durch einen Schaft nach Fig. 1, der von einem schematisch angedeuteten Zementbett umhüllt und in einen Knochen eingesetzt ist;

Fig. 3 stellt - gegen Fig. 2 nochmals vergrössert ein Detail des neuen Verankerungsschaftes im-Schnitt III-III von Fig. 2 dar.

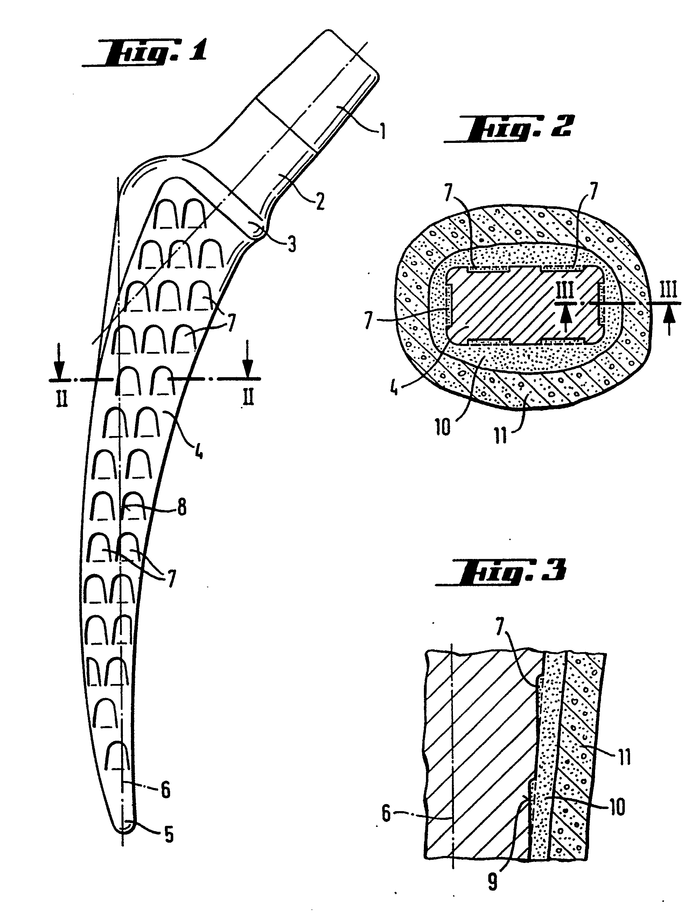

Zur Aufnahme des nicht gezeigten Gelenkkopfes dient bei der Femurkopfprothese nach Fig. 1 ein konischer Zapfen 1, der über einen prothesenhals 2 und elnen Kragen 3 in einen Verankerungsschaft 4 übergeht. Dieser Schaft 4, der einen rechteckigen Querschnitt hat (Fig. 2), weist eine leicht gekrümmte Form auf und verläuft vom Kragen 3 aus sich allseitig konisch verjüngend zu seinem distalen Ende 5. Seine Längsachse ist mit 6 bezeichnet.

Die Oberfläche des Schaftes 4 ist mit torbogenartigen Vertiefungen 7 versehen, die nach proximal konisch verlaufende Flanken 8 haben. Wie Fig. 3 zeigt, sind die Talsohlen 9 der Vertiefungen parallel zur Längsachse 6 ausgerichtet, so dass aufgrund der konischen Aussenform des Schaftes 4 die Vertiefungen 6 zum distalen Ende 5 hin stufenlos in die Schaftoberfläche übergehen.

Wie Fig. 1 erkennen lässt, können die Vertiefungen 7 beispielsweise in horizontalen Reihen angeordnet und zwischen den einzelnen Reihen schuppenartig zueinander versetzt sein. Ihre Höhen betragen etwa 5 mm, ihre Breite am Fuss der "Torbögen" etwa 3 bis 5 mm und ihre Tiefe weniger als 2 mm.

Beim Einführen des Schaftes in einen Knochenzement 10 (Fig. 2), der zuvor in einen operativ vorbereiteten Hohlraum des Femurknochens 11 eingefüllt worden ist, dringt der Knochenzement 10 in die Vertiefungen 7 ein; aufgrund der konischen Form der Flanken 8 verdichtet er sich dabei etwas. Beim Schwinden des Knochenzements 10 während und nach der Polymerisationsreaktion zieht sich der Ring des Knochenzements 10 sowohl in Umfangsrichtung als auch im Durchmesser etwas zusammen, wodurch eine gute Haftung in den Vertiefungen 7 erreicht wird.

**Patentansprüche**

1. Verankerungsschaft (4) für die Verankerung von Knochenimplantaten mit Hilfe von Knochenzement (10), welcher Schaft sich gegen sein freies Ende (5) allseitig und stetig verjüngt und mindestens auf Teilen der Schaftoberfläche mit einer Oberflächenstruktur versehen ist, dadurch gekennzeichnet, dass als Oberflächenstruktur eine Anzahl von torbogenartigen im wesentlichen in Richtung der Schaftlängsachse (6) verlaufenden Vertiefungen (7) mit sich von distal nach proximal konisch verjüngenden Seitenflanken (8) schuppenartig verteilt angeordnet ist.

2. Verankerungsschaft nach Anspruch 1, dadurch gekennzeichnet, dass die Vertiefungen (7) distal stufenlos in die Schaftoberfläche übergehen, und ihre Talsohlen (9) parallel zur Schaftlängsachse (6) verlaufen.

**Claims**

1. An anchorage stem (4) for anchoring bone implants with the use of bone cement (10), the stem narrowing towards its free end (5) on all sides and continuously and having at least on parts of the stem surface a surface structure or texture, characterised in that as surface structure or texture a number of arch-like recesses (7) which extend substantially in the direction of the longitudinal axis (6) of the stem and which have side flanks (8) narrowing conically from the distal in the proximal direction are distributed like scales.

2. A stem according to claim 1, characterised in that the recesses (7) merge steplessly on the distal side into the stem surface and their troughs (9) extend parallel to the stem longitudinal axis (6).

**Revendications**

1. Tige d'ancrage (4) pour la fixation d'implants osseux à l'aide d'ostéociment (10), cette tige accusant de toutes parts une décroissance de section continue en direction de son extrémité libre (5), et étant munie d'une structure superficielle au moins sur des parties de la surface de cette tige, caractérisée par le fait qu'un certain nombre de renfoncements arqués (7), s'étendant sensiblement dans la direction de l'axe longitudinal (6) de la tige, sont répartis à la manière d'écailles, en tant que structure superficielle, avec des flancs latéraux (8) se rétrécissant tronconiquement du côté distal vers le côté proximal.

2.Tige d'ancrage selon la revendication 1, caractérisée par le fait que les renfoncements (7) se prolongent sans interruption dans la surface de la tige du côté distal, leurs dépressions (9) s'étendant parallèlement à l'axe longitudinal (6) de cette tige.

**Fig. 1**

**Fig. 2**

**Fig. 3**